# EUROPÄISCHE PATENTSCHRIFT

(11) **EP 1 053 757 B1**
(45) Veröffentlichungstag und Bekanntmachung des Hinweises auf die Patenterteilung: **05.02.2003**
(21) Anmeldenummer: 00250142.7
(22) Anmeldetag: 12.05.2000
(51) Int. Cl.: A61L 27/38, A61L 27/60

(54) **Gewebekonstrukt für die Transplantationschirurgie**
Tissue scaffold for transplantation surgery
Construction cellulaire pour la chirurgie de transplantation

(30) Priorität: 15.05.1999 DE 19922078
(43) Veröffentlichungstag der Anmeldung: 22.11.2000
(73) Patentinhaber: Weitzel-Kage, Doris, 12207 Berlin (DE)
(72) Erfinder: Weitzel-Kage, Doris, 12207 Berlin (DE); Salomon, Andreas, 13503 Berlin (DE)
(74) Vertreter: Jungblut, Bernhard Jakob, Dr.

(56) Entgegenhaltungen:
- WO-A-98/40111
- WO-A-99/00152
- US-A- 5 770 448
- WAKI A ET AL: "Reassessment of FDG Uptake in Tumor Cells: High FDG Uptake as a Reflection of Oxygen-Independent Glycolysis Dominant Energy Production" NUCLEAR MEDICINE AND BIOLOGY,US,ELSEVIER SCIENCE PUBLISHERS, NEW YORK, NY, Bd. 24, Nr. 7, 1. Oktober 1997 (1997-10-01), Seiten 665-670, XP004092555 ISSN: 0969-8051

## Beschreibung

Die Erfindung betrifft ein Gewebekonstrukt für die Transplantationschirugie mit einer Sandwichstruktur enthaltend ein flächiges Substrat und eine auf dem flächigen Substrat anhaftende Schicht von kultivierten Körperoberflächenzellen. - Der Ausdruck Gewebekonstrukt meint einen Gegenstand, welcher lebende Zellen auf einem nicht-lebenden Substrat aufweist. Hierbei ist mit Gewebe nicht zwingend ein Zellverband, wie in einem Organismus vorkommend gemeint. Insbesondere bezeichnet der Ausdruck Gewebe im Rahmen der Erfindung nicht zwingend eine Zellansammlung, in welcher aufgrund des Zellwachstums bzw. der Zellproliferation eine Kontaktinhibierung in beachtlichem Maße bereits eingetreten ist.

Gewebekonstrukte werden insbesondere als gleichsam künstliche Haut zum Verschluß und zur Regeneration von Wunden, insbesondere Verbrennungswunden, verwendet. Eine Regeneration ist erforderlich, wenn eine größere Fläche der Epidermis und der Dermis bei einem Patienten fehlt, beispielsweise aufgrund der bei schweren Verbrennungen erforderlichen Excision der verbrannten Haut oder des Narbengewebes. Mit Regeneration ist die Ausbildung von neuer Epidermis und Dermis im Bereich der Fehlstelle gemeint. Gewebekonstrukte können zum Schließen anderer Defektwunden (angeboren, bei Durchblutungsstörungen oder anderen) verwendet werden.

Die Literaturstelle WO99/00152 offenbart ein biosynthetisches Hauttransplantat mit einer Kollagenmatrix, die mit autologen Keratinozyten besiedelt wird. Die Literaturstelle US 5 770 448 beschreibt ein Verfahren zur Verbesserung der Anwachssicherheit von Zellen auf Gewebekonstrukten durch eine Beschichtung mit extrazellulärer Matrix. Aus der Literaturstelle A. Waki et al., Nuclear Medicine & Biology, Vol. 24, pages 665-670 (1997) ist entnehmbar, daß Kontaktinhibierung bei Zellkulturen durch Wahl der Kultivierungsbedingungen vermeidbar bzw. steuerbar ist.

Gewebekonstrukte des eingangs genannten grundsätzlichen Aufbaus sind beispielsweise aus den Literaturstellen US-A 5,131,907, US-A 5,273,900 (hierin ist eine umfängliche Hintergrunddarstellung der technologischen Zusammenhänge gegeben), US-A 5,282,859, US-A 5,800,537 und US-A 5,888,248. Diesem Stand der Technik ist gemeinsam, daß als Substrat Collagen verwendet wird und daß die Körperoberflächenzellen zu einem dichten Zellrasen kultiviert sind, also Mehrschichtig und mit voll ausgebildeter Kontaktinhibierung. Es wird also ein vollständiges Gewebe (im eigentlichen Sinne des Ausdrucks) in vitro hergestellt. Die Verwendung von Collagen als Substrat hat mehrere Nachteile. Zum ersten ist Collagen, sei es unvernetzt oder vernetzt, nicht hinreichend elastisch bzw. nicht hinreichend elastisch dehnbar, um bei (dreidimensional) komplexen Wundenformen im gesamten Wundbereich anliegend eingebracht werden zu können. Ein vollständiges Anliegen ist jedoch eine wesentliche Voraussetzung für ein Anwachsen der Körperoberflächenzellen an das Gewebe des Patienten. Weiterhin ist Collagen opak bzw. milchig trüb, weshalb bei der Kultivierung der Körperoberflächenzellen auf dem Collagen eine lichtmikroskopische Betrachtung der Zellen erschwert ist. Insgesamt zeigt es sich, daß die insofern bekannten Gewebekonstrukte nicht hinreichend sicher anwachsen, wodurch im Falle des Nichtanwachsens wiederholte Behandlungen mit der kunstlichen Haut und/oder Transplantationen zwingend erforderlich werden.

Für biokompatible und ggf. auch resorbierbare Membranen und Materialien ist Collagen als solches vielfach beschrieben. Hinsichtlich vernetzten Collagens ist beispielsweise auf die Literaturstellen US-A 5,874,537, US-A 5,782,931 und US-A 5,795,605 zu verweisen. Aber auch vernetzte Gelatine ist als Werkstoff für biokompatible Membrane als solches bekannt. Hierzu wird beispielsweise auf die Literaturstellen US-A 5,834,232 und US-A 5,723,142 verwiesen.

Gegenüber den vorstehend beschriebenen Gewebekonstrukten liegt der Erfindung das technische Problem zugrunde, eine höhere Anwachssicherheit zu gewährleisten.

Zur Lösung diese Problems lehrt die Erfindung, daß auf dem Substrat, welches als resorbierbares Trägermaterial Gelatine aufweist, die Kultivierung der Körperoberflächenzellen mit der Maßgabe durchgeführt wird, daß zumindest 10% der Körperoberflächenzellen nicht kontaktinhibiert sind und daß das Substrat ein resorbierbares Trägermaterial mit einem Elastizitätsmodul von weniger als 1 N/mm² (18°C) aufweist. - Kontaktinhibition ist ein physiologischer Prozeß der Zellkommunikation, wobei die Detektion von Oberflächenmolekülen zweier benachbarter Zellen die Proliferation inhibiert. Hierdurch wird ein unkontrolliertes Wachstum auf natürliche Weise verhindert. Eine Zelle, welche kontaktinhibiert ist, kann folglich nicht mehr proliferieren im Gegensatz zu einer Zelle, die (noch) nicht kontaktinhibiert ist. Körperoberflächenzellen bezeichnet alle Zellspezies, die eine Oberfläche eines Organismus bilden, sei es eine äußere oder einen innere Oberfläche. Es versteht sich, daß die Körperoberflächenzellen mit der Maßgabe ausgewählt werden, daß sie für die zu ersetzende Oberfläche eines Patienten geeignet sind. Vorzugsweise werden Fehlstellen mit jeweils der gleichen Spezies an Körperoberflächenzellen repariert bzw. behandelt. Der wesentliche Bestandteil eines Substrats ist das Trägermaterial. In dem Trägermaterial oder auf einer Oberfläche des Trägermaterials können Stoffe vorgesehen sein, welche die Eigenschaften des Trägermaterials nicht wesentlich beeinflussen, aber von therapeutischem Nutzen oder der Zellkultivierung förderlich sind. Beispielhaft hierfür werden genannt: Antibiotika (z.B. Tetracycline), Chemotherapeutika (z.B. Taurolidin), Wachstumsfaktoren, Anaesthetika (lokal). Das Elastitizätsmodul gibt die Elastizität eines Werkstoffes an und ergibt sich aus dem Quotienten des Spannungsdifferentials (der Normalspannung) durch das Differential der Dehnung. Die Spannung ist der Quotient aus Kraft- und Flächendifferential. Die Dehnung ist die Längendifferenz dividiert durch die ursprüngliche Länge. Mit einem erfindungsgemäßen Konstrukt wird, mit anderen Worten ausgedrückt, neue Haut mit ihrer Schichtstruktur nicht in vitro sondern erst in vivo, also nach Aufbringen des Gewebekonstrukts, gebildet.

Mit der Erfindung wird eine überraschend hohe Sicherheit hinsichtlich des Anwachsens des Gewebekonstrukts in einer Fehlstelle erreicht. Nachträgliche Maßnahmen, insbesondere erneute Transplantationen sind in praktisch allen Fällen entbehrlich. Selbstverständlich kann aus Gründen der Sicherheit ein Wiederholungsgang durchgeführt werden. Die mit dem Gewebekonstrukt neu gebildete Oberflache, insbesondere Haut, wird gut durchblutet und bildet kein störendes Narbengewebe. Es wird eine kosmetisch in beachtlichem Maße zufriedenstellende Hautneubildung erreicht. Ohne an eine Theorie gebunden zu sein, wird vermutet, daß aufgrund nicht vorhandener Kontaktinhibierung das Gewebekonstrukt besonders gut in eventuelle Lücken hineinwachsen kann. Durch die elastischen Eigenschaften des Trägermaterials werden eventuelle Lücken zudem recht klein gehalten, da sich ein erfindungsgemäßes Gewebekonstrukt mit sogenanntem saugenden Kontakt einbringen läßt.

Je nach Anwendung ist es bevorzugt, wenn die Körperoberflächenzellen ausgewählt sind aus der Gruppe bestehend aus "Keratinozyten und Epithelzellen des Respirationstrakts, der Mundschleimhaut, der Cornea oder innerer Körperoberflächen wie Harnblase, Gallenblase und dergleichen". Falls das Gewebekonstrukt zum Ersatz zerstörter Haut verwendet werden soll, so ist der Einsatz von Keratinozyten bevorzugt. Es kann auch mit Gewebekonstrukten enthaltend Fettzellen oder Fibroblasten gearbeitet werden.

Bei der Regeneration von Haut können erfindungsgemäße Gewebekonstrukte mit Fettzellen und/oder Fibroblasten und/oder Keratinozyten in dieser Reihenfolge in zeitlichem Abstand (1 bis 5 Tage) und in der angegebenen Reihenfolge aufgebracht werden. Es ist aber im Rahmen der Erfindung auch möglich, ein Multilayersandwich enthaltend die Schichtfolge (von der Wunde aus gesehen) "Fettzellen, Substrat, Fibroblasten, Substrat, Keratinozyten, Substrat" herzustellen und aufzubringen. In letzteren Falle werden die Substratschichten nach der Applikation subsequent von der Wundenseite her aufgelöst aufgrund der in der Wunde vorliegenden Kollagenasen. Mit dem Einsatz von Fibroblasten wird die Ausbildung von neuem Bindegewebe gefördert. In beiden Alternativen dieses Ausführungsbeispiels können die Fettzellen oder die Fibroblasten weggelassen werden.

Hinsichtlich der Zelldichte der Körperoberflächenzellen ist es bevorzugt, daß zumindest 20%, vorzugsweise zumindest 50%, höchstvorzugsweise zumindest 70%, besser 80 oder 90 bis 100%, der Körperoberflächenzellen nicht kontaktinhibiert sind. Je höher der Anteil nicht kontaktinhibierter Körperoberflächenzellen ist, um so besser gelingt das Anwachsen des Gewebekonstrukts in einer Fehlstelle.

Hinsichtlich der mechanischen Eigenschaften ist es bevorzugt, wenn das Trägermaterial ein Elastizitätsmodul von weniger als 1 N/mm², vorzugsweise von weniger als 0,5 N/mm², höchstvorzugsweise im Bereich von 0,05 bis 0,15 N/mm² aufweist. Je elastischer das Trägermaterial ist, um so leichter läßt sich das Gewebekonstrukt in einer (dreidimensional) komplexen Fehlstelle vollständig zum Anliegen bringen. Die Bruchdehnung sollte möglichst hoch sein und zumindest oberhalb von 20%, besser oberhalb von 50% liegen. Die Bruchdehnung ist definiert als die Dehnung, bei welcher Bruch erfolgt. Es versteht sich, daß das Elastizitätsmodul nicht so klein sein darf, daß unter dem Eigengewicht des Gewebekonstrukts die Bruchdehnung erreicht wird. Die angegebenen Werte beziehen sich auf 18°C und Luftumgebung. Nach dem Einbringen eines erfindungsgemaßen Konstrukts in eine Wunde ändern sich die Materialeigenschaften. Typischerweise hat das Substrat eine Dicke von 0,1 bis 5 mm, vorzugsweise von 0,3 bis 2 mm, höchstvorzugsweise von 0,3 bis 1 mm.

In optischer Hinsicht ist es bevorzugt, wenn das Substrat transparent und optisch klar ist. Dann kann der Kultivierungsfortschritt ohne weiteres mit einem Inverslichtmikroskop beobachtet werden. Dies ist von durchaus wesentlicher praktischer Bedeutung, da dann die Kultivierung leicht überwacht und bei Erreichen einer gewünschten Zelldichte bzw. vor Erreichen einer unerwünscht hohen Kontaktinhibierung abgebrochen werden kann.

Ein allen mechanischen Anforderungen genügendes Gewebekonstrukt wird erhalten, wenn das Trägermaterial aus mittels eines Elastizität vermittelnden Vernetzungsmittels vernetzter Gelatine besteht. Gelatine ist ein Abbauprodukt des Collagens. Die Gelatine kann aus Collagenen verschiedener Typen, also aus Collagen I bis IV gewonnen werden. Das Collagen kann menschlicher oder tierischer Herkunft sein. Gut geeignet ist eine Gelatine aus Schweinehaut mit einer Bloom-Stärke von 100 bis 400, beispielsweise 300. Als Vernetzungsmittel kommen alle denkbaren Stoffe in Frage, sofern sie einerseits physiologisch unbedenklich und andererseits in ausreichendem Maße elastizitätsvermittelnd sind. In chemischer Hinsicht ist das Vernetzungsmittel bevorzugt eine organische Verbindung der Formel R1-X-R2 ist, wobei R1 und R2 gleich oder verschieden sein können und Gruppen darstellen, welche mit Aminogruppen und/oder Carboxylgruppen, ggf. auch mit Sulfhydrylgruppen, umsetzbar sind und wobei X C2-C20-Alkyl ggf. mit einer Elastizität vermittelnden funktionellen Gruppe ist. Für R1 und R2 sind beispielsweise zu nennen: -CHO, Imidoester eines C1 bis C5 Alkanols, N-Hydroxysuccinimidylester, Maleinimid, α-Haloacetal, Pyridyldisulfid. Als Beispiel einer Elastizität vermittelnden Gruppe ist Carbamat zu nennen. Bevorzugt sind homobifunktionelle Vernetzungsmittel, insbesondere 1,5-Pentandial.

Die Herstellung des Trägermaterials kann ausgehen von einer 7 bis 25%-igen (w/v), vorzugsweise einer 10 bis 20%-igen, höchstvorzugsweise einer 10 bis 14%-igen wäßrigen Gelatinelösung. Bei ca. 20% wird eine zwar geringfügig verminderte Elastizität erhalten, die Stabilität wird jedoch deutlich erhöht.

Ein erfindungsgemäßes Gewebekonstrukt ist dadurch erhältlich, daß die Schicht aus Körperoberflächenzellen durch Aufbringen von einzelnen Körperoberflächenzellen und Kultivierung der aufgebrachten Körperoberflächenzellen bis zur Ausbildung einer Schichtdicke von maximal einer Monoschicht aufgebaut wird.

Bevorzugt ist es, wenn die Kultivierung der Korperoberflächenzellen, beispielsweise der Keratinozyten, Fibroblasten und Fettzellen, zumindest homolog, vorzugsweise autolog durchgeführt wird. Homologe Kultivierung meint die Kultivierung in Serum eines anderen Individuums gleicher Spezies enthaltendem Medium. Autologe Kultivierung meint die Kultivierung in Serum des gleichen Individuums, für welches das Gewebekonstrukt bestimmt ist, enthaltendem Medium.

Homologe Kultivierung ist angebracht, wenn das Serum des Individuums, für welches das Gewebekonstrukt bestimmt ist, beispielsweise aufgrund von Medikation nicht verwendbar ist oder aufwendig behandelt werden müßte. Der Anteil an Serum im Medium beträgt typischerweise 1 bis 20%, beispielsweise 10%. Als Hauptbestandteil des Medium kommen übliche Mediumzusammensetzungen in Frage, wovon lediglich beispielhaft DMEM genannt wird. Grundsätzlich kann auch serumfrei gearbeitet werden.

Ein erfindungsgemäßes Gewebekonstrukt ist beispielsweise im einzelnen erhältlich durch die folgenden Verfahrenstufen: a) einem Organismus, vorzugsweise einem Patienten, für welchen das Gewebekonstrukt bestimmt ist, wird Körperoberflächenzellen enthaltendes Gewebe entnommen, b) das Gewebe aus Stufe a) wird mechanisch zerkleinert und die Körperoberflachenzellen werden aus dem zerkleinerten Gewebe isoliert, c) die Körperoberflächenzellen aus Stufe b) werden durch Passagieren, vorzugsweise mehrfaches Passagieren vermehrt, d) die in Stufe c) erhaltenen Körperoberflächenzellen werden mittels einer Protease, vorzugsweise mittels einer Endopeptidase, hochstvorzugsweise mittels Trypsin, vereinzelt und in einem vorzugsweise autologes Serum enthaltenden Medium suspendiert, e) die suspendierten Körperoberflächenzellen aus Stufe d) werden auf dem Substrat ausgesaht und kultiviert, wobei die Aussaatdichte und die Kultivierungszeit mit der Maßgabe eingerichtet sind, daß die Körperoberflächenzellen bei dem transplatationsfertigen Konstrukt eine Monolage bilden und zumindest 10% der Körperoberflächenzellen nicht kontaktinhibiert sind.

Mit Passagieren ist das enzymatische Ablösen (und Vereinzeln) von Zellen nach einer Kultivierung und erneutes Aussähen in einer niedrigen Zelldichte bezeichnet. Dadurch wird aus einer vergleichsweise kleinen Anzahl einer Person entnommener Zellen schnell eine hohe Anzahl von Zellen erhalten. Das Aussähen von Zellen erfolgt in aller Regel (sowohl beim Passagieren als auch beim Aussähen in Stufe e) als Ablage einer Zellsuspension in dem (serumhaltigen) Kultivierungsmedium. Im Falle von Keratinozyten ist in der Stufe e) eine Aussaat mit einer Dichte von 10³ bis 10⁵, vorzugsweise ca. 10⁴ Zellen/cm², in Verbindung mit einer Kultivierungs- dauer von 12 bis 48 Stunden, vorzugsweise 24 bis 36 Stunden, zweckmäßig. Bevorzugt ist es, wenn das Passagieren und/oder die Kultivierung in einem Medium enthaltend einen Stoff oder mehrere Stoffe aus der Gruppe bestehend aus "einfach ungesättigte Fettsäuren, mehrfach gesättigte Fettsäuren, Butyrate" erfolgt. Es hat sich gezeigt, daß sich dann ein besonders gutes Zellwachstum bzw. eine besonders gute Proliferation einstellt. Als einsetzbare Fettsäuren werden beispielhaft genannt: Crotonsäure, Palmitoleinsäure, Ölsaure, Erucasaure, Sorbinsäure, Linolsäure, Linolensäure, Elaeostearinsäure, Arachidonsäure, Clupanodonsäure und Docosahexaensäure. Butyrate sind Salze und Ester, beispielsweise C1 bis C10 Alkylester, der Buttersäure. Als Salzbildner ist in jedem Fall Natrium bevorzugt.

Das Gewebekonstrukt sollte möglichst homogen hinsichtlich der Körperoberflachenzellen sein. Dies gelingt besonders gut, wenn die Aussaat in Stufe e) durch Auflage eines sterilen Gitters auf das Substrat und Einbringung der Suspension in die Gittersegmente erfolgt, wobei das Gitter nach Sedimentation und Anhaftung der Körperöberflächenzellen entfernt wird. Das Gitter hat mindestens ein Rastermaß von 1 mm, zweckmäßigerweise 1/10 bis 1/100 der Kantenlänge eines Substrats. Die Aussaat kann mehrfach erfolgen. Dann ist die Gesamtmenge der ausgesähten Körperoberflächenzellen gegenüber einer einmaligen Aussaat unverändert. Mit anderen Worten ausgedrückt bei beispielsweise dreimaliger Aussaat wird bei jeder Aussaat ein Drittel der auszusähenden Gesamtmenge an Körperoberflächenzellen ausgesäht.

Im Rahmen der Kultivierung in Stufe b) - e) kann ohne Feederlayer (Zellschicht, die nicht von der zu behandelnden Person stammt, vor der Aussaat der Körperoberflächenzellen kultiviert und auf geeignete Weise inaktiviert wird) und/oder ohne tierische Seren oder Gewebeextrakte gearbeitet werden.

Die Erfindung betrifft weiterhin die Verwendung eines erfindungsgemäßen Gewebekonstrukts zum Ersatz von schädigungs- oder fehlfunktionsbedingt excisierter Haut, insbesondere von verbrennungsbedingt excisierter Haut.

Die Erfindung kann im Rahmen des folgenden Heilverfahren zum Einsatz kommen. Zunächst wird der Wundgrund des Hautdefekts in üblicher Weise vorbereitet, insbesondere wird zerstörtes (oder vernarbtes/sonstwie defektes) Hautgewebe (z.B. einschließlich des Fettgewebes) entfernt. Dann wird das erfindungsgemäße Gewebekonstrukt mit der Zellschicht in Richtung auf den Wundgrund auf- bzw. eingebracht. Gegebenenfalls kann das Gewebekonstrukt zuvor in geeigneter Weise zugeschnitten werden. Dann wird ein Verband angebracht, welcher so ausgeführt ist, daß der damit auf das Gewebekonstrukt ausgeübte Anpressdruck möglichst gleichmäßig ist. Nach einigen Tagen ist das Substrat durch Collagenasen der Keratinozyten und/oder des Wundsekrets (z.B. Granulozyten) vollständig degradiert.

Im Folgenden wird die Erfindung anhand von lediglich Ausführungsformen darstellenden Beispielen näher erläutert.

### Beispiel 1: Vorbereitung der Gießkassetten.

Die Gießküvette, bestehend aus zwei Glasplatten und einem Spacer aus Gummi, der nach Maßgabe der einzustellenden Dicke des Substrats ausgewählt wird, wird vor dem Zusammensetzen gründlich mit Wasser und Spülmittel gereinigt und dann gründlich mit destilliertem Wasser gespült. Die Glasplatten und der Spacer werden dann unter einer Sterilwerkbank mindestens dreimal mit 70% abs. ETOH in Wasser und einem sterilen Mulltuch gründlich abgerieben. Die Gießküvette wird dann zusammengesetzt, wobei die Oberseite nicht durch den Spacer abgedichtet wird und stattdessen mit einem sterilen Klebeband verschließbar ist. Nach dem Zusammenbau wird die Gießküvette in einem Wärmeschrank bei 60°C für mindestens 30 min. erwärmt.

### Beispiel 2: Lösen der Gelatine.

Zur Herstellung des Substrats wird eine 12% (w/v) wäßrige Gelatinelösung verwendet. Hierzu wird in ein durch Autoklavieren sterilisiertes Becherglas die entsprechende Menge an sterilem, pyrogenfreiem Wasser ("aqua ad injectabilia") eingefüllt und die Gelatine (gewonnen aus Schweinehaut, mit einer Bloom-Stärke von 300) bei Zimmertemperatur unter Rühren (beispielsweise mittels Magnetrührer) dem Wasser beigemengt. Es entsteht eine hochviskose Suspension. Der pH-Wert der Suspension wird auf pH 7,6 bis 7,8 eingestellt. Das Becherglas wird beispielsweise mit Aluminiumfolie verschlossen und die Suspension erwärmt, wobei die Gelatine gelöst wird. Hierbei sollte ein langeres Aufkochen vermieden werden. Die Erwärmung kann beispielsweise mittels Microwelle erfolgen.

### Beispiel 3: Gießen der Membran.

Die heiße Gelatinelösung wird mittels Rühren homogenisiert, in einer sterilen Spritze aufgezogen und in die vorgewärmte Gießküvette eingefüllt. Nach dem Einfüllen verbleibt die Gießkuvette noch für ca. 15 min. in Wärmeschrank (60°C), wobei sich eventuelle Luftblasen absetzen. Danach wird die Gelatine bei 4°C für mindestens 10 Stunden polymerisiert.

### Beispiel 4: Vernetzen der Gelatine.

Das Substrat wird unter einer sterilen Werkbank aus der Gießküvette herausgelöst und in eine 4°C kalte wäßrige ("aqua ad injectabilia") Lösung mit 0,0083 Vol.-% 1,5-Pentandial für eine Dauer von 12 Stunden gelegt. Grundsätzlich kann mit Konzentrationen an 1,5-Pentandial von 0,001 bis 0,05 Vol.-% gearbeitet werden, wobei bei höherer Konzentration mit kürzeren Vernetzungszeiten (bei 0,0125 Vol.-% beispielsweise 30 Minuten) gearbeitet werden sollte. Anschließend wird die 1,5-Pentandial-Lösung entfernt und das Substrat zweimal für 60 Minuten mit sterilem pyrogenfreiem Wasser ("aqua ad injectabilia") gewaschen. Das Substrat wird dann bis zur weiteren Verwendung in 70% abs. ETOH gelagert. Alternativ kann das Substrat mit 0,5% Glycerin gewaschen, gefriergetrocknet und steril verpackt werden.

### Beipiel 5: Rehydratisierung des Substrats.

Das Substrat wird aus dem Alkohol (oder der Sterilverpackung) genommen und zweimal für 60 Minuten mit steriler 0,9% NaCl-Lösung rehydratisiert. Im Zuge des Rehydratisierens können Zusatzstoffe wie Wachstumsfaktoren (z.B. EGF), Differenzierungsfaktoren oder Antibiotika in das Substrat eingebracht werden. Das so erhaltene Substrat ist fertig für die Aussaat von Zellen.

### Beispiel 6: Alternative Substratherstellung.

Alternativ zu den Beispielen 1 bis 5 kann mit einem Spacer bzw. einem Gefäß gearbeitet werden, dessen Dicke ein Vielfaches der für das Substrat einzustellenden Dicke aufweist. Das Substrat kann dann durch Abschneiden von dem so erhaltenen Gelatineblock hergestellt werden. Das Vernetzen kann vor oder nach dem Abschneiden erfolgen.

### Beispiel 7: Messung des Elastizitätsmoduls

Ein Substrat der Abmessungen 131 mm x 105 mm 0,5 mm wurde an einer Seite (131 mm x 0,5 mm) befestigt. Die gegenüberliegende Seite wurde mit Gewichten kraftbeaufschlagt. Es ergaben sich die folgenden Werte:

| Masse (g) | Länge (mm) | Dehnung | E (N/mm²) |
|---|---|---|---|
| 200 | 145 | 0,381 | 0,081 |
| 280 | 150 | 0,429 | 0,100 |
| 490 | 180 | 0,714 | 0,106 |

Nach Entlastung erfolgt Rückstellung auf die Ursprungslänge innerhalb von hochstens ca. 60 Sekunden.

### Beispiel 8: Aufbringen der Keratinozyten auf das Substrat.

Die Keratinozyten werden aus dem mechanisch zerkleinerten Gewebe auf übliche Weise isoliert und vorkultiviert. Dies erfolgt durch mehrfaches Passagieren der Zellen. Dabei werden die Zellen enzymatisch von der Unterlage abgelöst und anschließend in einer niedrigen Zelldichte ausgesäht. Durch Vermehrung der Zellen steigt die Zelldichte nachfolgend an, die Zellen können ggf. mehrlagig wachsen.

Vor der Aussaat auf dem Substrat werden die Keratinozyten konditioniert. Dies erfolgt durch ein Ablösen von der Unterlage mittels Trypsin. Anschließend werden die Zellen auf dem Substrat (0,3 bis 1 mm Dicke) ausgesäht. Die Aussaatdichte und nachfolgende Kultivierungszeit wird so gewählt, daß die Zellen bei dem Einsatz des Gewebekonstrukts einlagig dicht liegen, ihre Vermehrung jedoch nicht durch Kontaktinhibition wesentlich reduziert ist, beispielsweise 10⁴ Zellen/cm² mit nachfolgender Kultivierung über 24 bis 36 Stunden. Die Aussaat erfolgt mittels eines sterilen Gitters, welches auf das Substrat aufgelegt wird. In die Gittersegmente wird die Suspension von Zellen in Serum-haltigen Kulturmedium (DMEM) einpipettiert. Nach Sedimentation und Anhaften der Zellen am Substrat wird das Gitter wieder entfernt (nach 30 bis 60 Minuten). Dies kann mehrfach wiederholt werden.

Die Kulturbedingungen sind wie folgend: keine Feederlayer (Zellschicht zur Unterstützung des Wachstums der Keratinozyten); autologe, ggf. homologe Bedingungen; kein Einsatz tierischer Seren oder Gewebeextrakte; Zugabe von 1 bis 50 µg/ml einer Fettsäure, beispielsweise 5 µg/ml Na-Linolsäure, zum Medium.

### Beipiel 9: Durchführung der Transplantation.

Ein erfindungsgemäßes Gewebekonstrukt wird auf die Oberfläche des Hautdefekts nach geeigneter Vorbereitung des Wundgrundes mit der Zellschicht in Richtung auf den Wundgrund (gleichsam einem Abziehbild) aufgebracht, ggf. nach Zuschnitt des Gewebekonstrukts. Bei tieferen Defekten kann mit vergleichsweise dicken Substraten gearbeitet werden. Dann erfolgt ein Verband, welcher das Gewebekonstrukt möglichst gleichmäßig druckbeaufschlagt. Nach wenigen Tagen ist die Zellschicht angewachsen und das Substrat degradiert. Die Zellschicht wächst nach praktisch jeder Transplatation an und bildet ein narbenfreies und gut durchblutetes neues Körpergewebe. Aus Sicherheitsgründen kann eine weitere Transplantation nachgeschaltet werden, ist jedoch nicht zwingend erforderlich.

### Beispiel 10: Quantifizierung des Anteils kontaktinhibierter Zellen.

Kontaktinhibition von Keratinozyten ist morphologisch gekennzeichnet durch die Ausbildung von sehr engen Zell-Zell-Kontakten, den tight junctions, die durch membranständige cell adhesion molecules vermittelt werden. Diese durch hochauflösende optische Verfahren gut darstellbaren Zellkontakte sind eine wesentliche charakteristische Eigenschaft von Keratinozyten. Intrazellulär wird durch die Kontaktinhibition eine Reihe von bisher nicht vollständig aufgeklärten Mechanismen induziert, die die Zellen von einem proliferativen in einen sekretorischen Zustand überführen. Insbesondere bei Keratinozyten findet auch eine Veränderung der Zellform und Oberfläche statt. Grundsätzlich sind demgemäß Marker des Übergangs von der proliferativen zum sekretorischen Zustand sowie die Bestimmung von tight junction-bildenden Oberflächenproteinen oder eine direkte hochauflösende optische Bestimmung des Vorliegens von tight junctions geeignet, das Ausmaß der Kontaktinhibition in einer Zellkultur zu bestimmen.

Durch optische Betrachtung und Auszählung der Anzahl der tight junctions einer Zelle kann daher definiert werden, ob die betreffende Zelle kontaktinhibiert ist der nicht. Ist die Anzahl der tight junctions, welche einer Zelle zuzuordnen sind, geringer als 4, so qualifiziert die Zelle als nicht kontaktinhibiert. Ist die Anzahl der tight junctions oberhalb von 10, so qualifiziert die Zelle als kontaktinhibiert. Zellen mit 4 bis 10 tight junctions bleiben unberücksichtigt. Wenn mit diesen Maßgaben eine Zellgruppe von beispielsweise 100 Zellen, besser 1000 Zellen, ausgezählt wird, so kann ohne weiteres der Anteil der nicht kontaktinhibierten Zellen bestimmt werden.

Die im Rahmen der Kontaktinhibition entstehenden Zell-Zell-Verbindungen haben jedoch noch einen weiteren Effekt. Durch die Einbindung in einen Zellverband reduziert sich wesentlich die Migrationsfähigkeit der betroffenen Zellen. Dies gilt insbesondere für Keratinozyten. Daher kann die Kontaktinhibition durch Bestimmung der Migrationsfähigkeit in einem Migrationstest bestimmt werden. Ein Migrationstest kann wie folgt durchgeführt werden.

Zur Prüfung der Kontaktinhibition werden Keratinozytenkulturen mikroskopisch mit einer Zeitkinetik in verschiedenen optischen Ebenen (z.B. mit Hilfe eines konfokalen Lasermikroskops) über eine definierte Zeit, typischerweise zwischen 2-24 Stunden, vorzugsweise 6 bis 10 Stunden, beispielsweise 8 Sunden, erfaßt. Anschließend wird die Zahl der innerhalb des Zeitraums gewanderten Zellen gezählt. Als Bewegung einer Zelle wird gewertet, wenn sich die betreffende Zelle innerhalb der Beobachtungszeit um einen Weg größer als der größte Zelldurchmesser (der betreffenden Zelle) in einer Ebene orthogonal zur optischen Achse des Mikroskops bewegt hat. Zur besseren Identifizierung und Abgrenzung können die Zellen mit einem membranständigen vitalen Farbstoff (z.B. PHK2-GL, erhältlich von der Firma Sigma) markiert werden.

Das vorstehende allgemeine Vorgehen ist ohne weiteres anwendbar für eine weitgehend homogene Keratinozytenkultur mit einem Anteil von Nicht-Keratinozyten (z.B. Fibroblasten) von weniger als 10%. Bei einem höheren Gehalt fremder Zellen müssen diese vor der Zählung durch spezifische Marker kenntlich gemacht und bei der Auswertung ausgelassen werden. Die Verteilung der Zelltypen wird gegebenenfalls nach Ausdifferenzierung der Zellen und Färbung mit geeigneten spezifischen Markern bestimmt.

Konkret wird wie folgt gemessen. In einer 25cm²-Zellkulturflasche werden unter dem Mikroskop zu untersuchende Regionen markiert. An jeder markierten Stelle wird mit dem Skalpell ein feines Kreuz auf die Zellkulturflaschenunterseite geritzt. Zur Wiederfindung der markierten Region wird die Außenseite des Kreuzes mit einem Punkt versehen. Zum Fotografieren der Zellen wird das Kreuz unter dem Mikroskop bei einer 40-fachen Vergrößerung mittig in den Bildausschnitt gebracht und mit Hilfe des Punktes die untere Ebene scharf gestellt. Nach der Scharfstellung des Punktes wurden zwei volle Umdrehungen des Feintriebs vollzogen und das erste Foto (Kleinbild 24x36mm) gemacht. Anschließend wurde der Feintrieb fünfmal um eine halbe Umdrehung weiter gedreht und jedes Mal ein Foto erstellt. Die Fotos werden mit einer 100-fachen Vergrößerung wiederholt. Danach werden die Fotos entwickelt und die Zellbewegungen mit Hilfe eines feinen Rasters, das anhand des Kreuzes auf den Fotos genau positioniert werden kann, beobachtet. Zur Ermittlung der Zellbewegungen wird auf den 9 x 13 cm Fotos ein 4 x 4 cm großes durchsichtiges Raster aufgelegt und die Zellzahl in diesem 16 cm² großen Feld ermittelt. Dieses Feld entspricht einer realen Größe von 2.89 mm². Die Zellpositionen werden auf dem Raster markiert und mit einem Bild, das 8 Stunden später aufgenommen wird, verglichen. Die Auszählung und Auswertung erfolgt nach der vorstehenden Definition, wonach eine Zelle nicht kontaktinhibiert ist, welche in dem Referenzzeitintervall um eine Wegstrecke gewandert ist, welche größer als der größte Durchmesser der betreffenden Zelle (gemessen am Ende des Zeitintervalls) ist.

Es zeigt sich generell eine Abnahme der Zellbewegungen mit zunehmender Zellzahl. Bei hohen Zellzahlen sind in definierten Bereichen keine Zellwanderungen mehr zu erkennen sondern nur noch geringfügige Zellbewegungen.
Zur besseren Darstellung der einzelnen Zellen in einem dichten Zellverband kann der Fluoreszenzfarbstoff PKH2-GL (Sigma) verwendet werden. Hierbei handelt es sich um einen aliphatischen Farbstoff der sich in die Zellmembran einlagert ohne eine toxische Wirkung auf die Zelle zu haben. Der Farbstoff ist für mehrere Tage in der Zellwand stabil. Die gefärbten Zellen können dann unter dem Fluoreszenzmikroskop beobachtet werden, wobei die Zellen sich dann nur noch als fluoreszierende Punkte darstellen. Zur Ermittlung der Mitoserate der Zellen können die PHK2-GL gefärbten Zellen mit Hilfe eines Durchflußzytometers (FACS) quantitativ gezählt werden. Hierbei halbiert sich bei jeder Zellteilung die Fluoreszenzintensität der Zellen, so dass alle Mitosen innerhalb eines definierten Zeitraums erfasst werden können.

## Patentansprüche

1. Gewebekonstrukt für die Transplantationschirugie mit einer Sandwichstruktur enthaltend ein flächiges Substrat und eine auf dem flächigen Substrat anhaftende Schicht von kultivierten Körperoberflächenzellen,
dadurch erhältlich,
daß auf dem Substrat, welches als resorbierbares Trägermaterial Gelatine aufweist,
die Kultivierung der Körperoberflächenzellen mit der Maßgabe durchgeführt wird, daß zumindest 10% der Körperoberflächenzellen nicht kontaktinhibiert sind.

2. Gewebekonstrukt nach Anspruch 1, wobei die Körperoberflächenzellen ausgewählt sind aus der Gruppe bestehend aus "Keratinozyten und Epithelzellen des Respirationstrakts, der Mundschleimhaut, der Cornea oder innerer Körperoberflächen wie Harnblase, Gallenblase und dergleichen".

3. Gewebekonstrukt nach Anspruch 1 oder 2, wobei die Sandwichstruktur ein Multilayersandwich enthaltend die Schichtfolge "Fettzellen, Substrat, Fibroblasten, Substrat, Keratinozyten, Substrat" ist.

4. Gewebekonstrukt nach einem der Ansprüche 1 bis 3, wobei zumindest 20%, vorzugsweise zumindest 50%, höchstvorzugsweise 80 - 100% der Körperoberflächenzellen nicht kontaktinhibiert sind.

5. Gewebekonstrukt nach einem der Ansprüche 1 bis 4, wobei das Trägermaterial ein Elastizitätsmodul von weniger als 1 N/mm², vorzugsweise von weniger als 0,5 N/mm² aufweist.

6. Gewebekonstrukt nach einem der Ansprüche 1 bis 5, wobei das Substrat transparent und optisch klar ist.

7. Gewebekonstrukt nach einem der Ansprüche 1 bis 6, wobei das Trägermaterial aus mittels eines Elastizität vermittelnden Vernetzungsmittels vernetzter Gelatine besteht.

8. Gewebekonstrukt nach einem der Ansprüche 1 bis 7, wobei das Vernetzungsmittel eine organische Verbindung der Formel R1-X-R2 ist, wobei R1 und R2 gleich oder verschieden sein können und Gruppen darstellen, welche mit Aminogruppen und/oder Carboxylgruppen umsetzbar sind, und wobei X C2-C20-Alkyl ggf. mit einer Elastizität vermittelnden funktionellen Gruppe ist.

9. Gewebekonstrukt nach einem der Ansprüche 1 bis 8, wobei die Kultivierung der Körperoberflächenzellen bzw. ggf. Fibroblasten zumindest homolog, vorzugsweise autolog durchgeführt wird.

10. Gewebekonstrukt nach Anspruch 9, welches erhältlich ist durch die folgenden Verfahrenstufen:
a) einem Organismus, vorzugsweise einem Patienten, für welchen das Gewebekonstrukt bestimmt ist, wird Körperoberflächenzellen enthaltendes Gewebe entnommen,
b) das Gewebe aus Stufe a) wird mechanisch zerkleinert und die Körperoberflächenzellen werden aus dem zerkleinerten Gewebe isoliert,
c) die Körperoberflächenzellen aus Stufe b) werden durch Passagieren, vorzugsweise mehrfaches Passagieren vermehrt,
d) die in Stufe c) erhaltenen Körperoberflächenzellen werden mittels einer Protease, vorzugsweise mittels einer Endopeptidase, höchstvorzugsweise mittels Trypsin, vereinzelt und in einem vorzugsweise autologes Serum enthaltenden Medium suspendiert,
e) die suspendierten Körperoberflächenzellen aus Stufe d) werden auf dem Substrat ausgesäht und kultiviert, wobei die Aussaatdichte und die Kultivierungszeit mit der Maßgabe eingerichtet sind, daß die Körperoberflächenzellen bei dem transplatationsfertigen Konstrukt eine Monolage bilden und zumindest 10% der Körperoberflächenzellen nicht kontaktinhibiert sind.

11. Gewebekonstrukt nach einem der Ansprüche 9 bis 10, wobei das Passagieren und/oder die Kultivierung in einem Medium enthaltend einen Stoff oder mehrere Stoffe aus der Gruppe bestehend aus "einfach ungesättigte Fettsäuren, mehrfach gesättigte Fettsäuren, Butyrate" erfolgt.

12. Gewebekonstrukt nach einem der Ansprüche 9 bis 11, wobei die Aussaat in Stufe e) durch Auflage eines sterilen Gitters auf das Substrat und Einbringung der Suspension in die Gittersegmente erfolgt, wobei das Gitter nach Sedimentation und Anhaftung der Körperöberflächenzellen entfernt wird.

13. Gewebekonstrukt nach Anspruch 12, wobei die Aussaat mehrfach erfolgt und wobei die Gesamtmenge der ausgesähten Körperoberflächenzellen gegenüber einer einmaligen Aussaat unverändert ist.

14. Gewebekonstrukt nach einem der Ansprüche 9 bis 13, wobei im Rahmen der Kultivierung ohne Feederlayer und/oder ohne Zusatz von tierischen Seren oder Gewebeextrakte gearbeitet wird.

15. Gewebekonstrukt nach einem der Ansprüche 1 bis 14 wobei die Schicht aus Körperoberflächenzellen durch Aufbringen von einzelnen Körperoberflächenzellen und Kultivierung der aufgebrachten Körperoberflächenzellen bis zur Ausbildung einer Schichtdicke von maximal einer Monoschicht aufgebaut werden.

16. Verwendung eines Gewebekonstrukts nach einem der Ansprüche 1 bis 15 zur Herstellung eines Transplantats zum Ersatz von schädigungs- oder fehlfunktionsbedingt excisierter Haut, insbesondere von verbrennungsbedingt excisierter Haut.

## Claims

1. A tissue construct for use in the transplantation field, having a sandwich structure comprising an areal substrate and a layer of cultivated body surface cells adhering to said areal substrate, obtainable by that on the substrate comprising gelatin as a resorbable carrier material, the cultivation of the body surface cells is performed such that at least 10 % of the body surface cells are not contact-inhibited.

2. A tissue construct according to claim 1, wherein the body surface cells are selected from the group consisting of "keratinocytes and epithelium cells of the respiratory tract, the mucous membrane, the cornea or other inner body surfaces such as the urine bladder, the gall bladder or the like.

3. A tissue construct according to claim 1 or 2, wherein the sandwich structure is a multilayer sandwich including the layer sequence "fat cells, substrate, fibroblasts, substrate, keratinozytes, substrate".

4. A tissue construct according to one of claims 1 to 3, wherein at least 20 %, preferably at least 50 %, most preferably 80 - 100 % of the body surface cells are not contact-inhibited.

5. A tissue construct according to one of claims 1 to 4, wherein the carrier material has a modulus of elasticity of less than 1 N/mm², preferably of less than 0,5 N/mm².

6. A tissue construct according to one of claims 1 to 5, wherein the substrate is transparent and optically clear.

7. A tissue construct according to one of claims 1 to 6, wherein the carrier material consists of gelatin cross-linked by means of a cross-linking agent providing elasticity.

8. A tissue construct according to one of claims 1 to 7, wherein the cross-linking agent is an organic composition of the formula R1-X-R2, wherein the groups R1 and R2 are identical or different and represent groups capable to be reacted with amino groups and/or carboxyl groups, and wherein X is C2-C20 alkyl, optionally with a functional group providing elasticity.

9. A tissue construct according to one of claims 1 to 8, wherein the cultivation of the body surface cells or if applicable fibroblasts is made at least in an homologous manner, preferably in an autologous manner.

10. A tissue construct according to claim 9, which is obtainable by the following steps:
a) from an organism, preferably a patient for whom the tissue construct is intended, tissue containing body surface cells are taken,
b) the tissue obtained in step a) is mechanically disintegrated, and the body surface cells are isolated from the disintegrated tissue,
c) the body surface cells of step b) are multiplied by passaging, preferably multiple passaging,
d) the body surface cells obtained in step c) are individualized by means of a protease, preferably by means of endopeptidase, most preferably by means of trypsin, and are suspended in a medium preferably containing autologous serum,
e) the suspended body surface cells of step d) are sown and cultivated on the substrate, the sow density and the cultivation time being selected such that the body surface cells in the construct ready to be transplanted form a mono-layer, and at least 10 % of the body surface cells are not contact-inhibited.

11. A tissue construct according to one of claims 9 to 10, wherein the passaging and/or cultivation takes place in a medium containing one substance or several substances of the group consisting of "singly unsaturated fatty acids, multiply saturated fatty acids, butyrates".

12. A tissue construct according to one of claims 9 to 11, wherein sowing in step e) is made by placing a sterile grid on the substrate and by bringing the suspension into the grid segments, the grid being removed after sedimentation and adhesion of the body surface cells.

13. A tissue construct according to claim 12, wherein sowing is performed several times, and the total quantity of the sown body surface cells is unchanged with regard to single sowing.

14. A tissue construct according to one of claims 9 to 13, wherein during cultivation, no feeder layer is used and/or no animal sera or tissue extracts are added.

15. A tissue construct according to one of claims 1 to 14, wherein the layer of body surface cells is built up by application of individual body surface cells and cultivation of the applied body surface cells up to a layer thickness of maximum one mono-layer.

16. Use of a tissue construct according to one of claims 1 to 15 for the production of a transplant for the replacement of skin excised by damage or malfunction, in particular excised due to burning.

## Revendications

1. Ensemble de tissu pour la chirurgie de la greffe avec une structure sandwich comprenant un substrat plan et une couche de cellules de surface du corps, que l'on peut obtenir par que sur le substrat comprenant comme porteur de la gélatine résorbable, la culture des cellules de surface du corps est exécutée de telle manière, qu'au moins 10 % des cellules de surface du corps ne soient pas inhibées par contact.

2. Ensemble de tissu selon la revendication 1, dans lequel les cellules de surface du corps sont choisies du groupe consistant en «kératinocytes et cellules épithéliales des organes de respiration, de la muqueuse orale, de la cornée ou des cellules de surface du corps internes comme p.ex. la vessie, la vésicule biliaire ou semblables».

3. Ensemble de tissu selon la revendication 1 ou 2, dans lequel la structure sandwich est un sandwich à multiples couches, comprenant la séquence des couches «cellules adipeuses, substrat, fibroblastes, substrat, kératinocytes, substrat».

4. Ensemble de tissu selon une des revendications 1 à 3, dans lequel au moins 20 %, de préférence au moins 50 %, de préférence la plus haute 80 à 100 % des cellules de surface du corps ne sont pas inhibées par contact.

5. Ensemble de tissu selon une des revendications 1 à 4, dans lequel le porteur a un module d'élasticité de moins que 1 N/mm², de préférence de moins que 0,5 N/mm².

6. Ensemble de tissu selon une des revendications 1 à 5, dans lequel le substrat est transparent et optiquement clair.

7. Ensemble de tissu selon une des revendications 1 à 6, dans lequel le porteur consiste en gélatine réticulée par un agent de réticulation établissant l'élasticité.

8. Ensemble de tissu selon une des revendications 1 à 7, dans lequel l'agent de réticulation est un composé organique de la formule R1-X-R2, R1 et R2 pouvant être identiques ou différents et représentant des groupes pouvant être réagis avec des groupes amino et/ou carboxyle, et X représentant C2-C20 alkyle, le cas échéant avec un groupe fonctionnel établissant l'élasticité.

9. Ensemble de tissu selon une des revendications 1 à 8, dans lequel la culture des cellules de surface du corps ou le cas échéant de fibroblastes se fait au moins en manière homologue, de préférence en manière autologue.

10. Ensemble de tissu selon la revendication 9, que l'on peut obtenir par les étapes suivantes:
a) du tissu comprenant des cellules de surface du corps est prélevé de l'organisme, de préférence d'un patient pour qui l'ensemble de tissu est prévu,
b) le tissu de l'étape a) est fractionné mécaniquement, et les cellules de surface du corps sont isolées du tissu fractionné,
c) les cellules de surface du corps de l'étape b) sont multipliées par passage, de préférence passage multiple,
d) les cellules de surface du corps obtenues dans l'étape c) sont individualisées au moyen d'une protéase, de préférence au moyen d'une endopeptidase, de préférence la plus haute au moyen de trypsine, et sont suspendues dans un milieu de préférence comprenant un sérum autologue,
e) les cellules de surface du corps suspendues de l'étape d) sont semées et cultivées sur le substrat, la densité des semailles et le temps de culture étant établis d'une telle manière que les cellules de surface du corps forment dans l'ensemble prêt à la greffe une mono-couche et qu'au moins 10 % des cellules de surface du corps ne soient pas inhibées par contact.

11. Ensemble de tissu selon une des revendications 9 à 10, dans lequel le passage et/ou la culture se fait dans un milieu comprenant une substance ou plusieurs substances du groupe consistant en «acides gras monosaturés, acides gras polysaturés, butyrates».

12. Ensemble de tissu selon une des revendications 9 à 11, dans lequel les semailles se font dans l'étape e) par application d'une grille stérile sur le substrat et introduction de la suspension dans les segments de la grille, la grille étant enlevée après la sédimentation et adhérence des cellules de surface du corps.

13. Ensemble de tissu selon la revendication 12, dans lequel les semailles se font plusieurs fois et la quantité totale des cellules de surface du corps semées est inchangée en comparaison avec des semailles uniques.

14. Ensemble de tissu selon une des revendications 9 à 13, dans lequel en ce qui concerne la culture, on travaille sans une couche feeder et/ou sans addition de sérums animaux ou extraits de tissu.

15. Ensemble de tissu selon une des revendications 1 à 14, dans lequel la couche de cellules de surface du corps est construite par l'application de cellules de surface du corps individuelles et culture des cellules de surface du corps appliquées jusqu'à une génération d'une épaisseur de couche d'au maximum une mono-couche.

16. Application d'un ensemble de tissu selon une des revendications 1 à 15 pour la production d'un greffon pour le remplacement de peau excisé en manière nuisible ou par fonction fautive, en particulier peau excisée par la brûlure.
